# EUROPEAN PATENT SPECIFICATION

(11) **EP 0 955 359 B1**
(45) Date of publication and mention of the grant of the patent: **07.01.2009**
(21) Application number: 99103453.9
(22) Date of filing: 11.06.1993
(51) Int. Cl.: C12N 5/00, A61M 35/00, C12N 5/10, A61K 48/00, C12N 15/86

(54) **Method for introducing genetic material into a cell**
Methode zur Einführung von genetischem Material in Zellen
Méthode d'introduction de matière génétique dans des cellules

(30) Priority: 11.06.1992 US 897357
(43) Date of publication of application: 10.11.1999
(62) Divisional of application: 93915336.7
(73) Proprietor: Elof Eriksson, Wellesley Hills, Massachusetts 02181 (US)
(72) Inventor: Eriksson, Elof, Wellesley Hills, Massachusetts 02181 (US); Vogt, Peter M., PD., 44789 - Bochum (DE)
(74) Representative: Duckworth, Timothy John

(56) References cited:
- WO-A-87/00201
- WO-A-91/08793
- E. ERIKSSON ET AL.: "In vivo cell culture accelerates reepitheliazation." SURGICAL FORUM, vol. 42, 1991, pages 670-673, XP000606925 Chicago, IL, USA
- E. FENJVES ET AL.: "Systemic distribution of apolipoprotein E secreted by grafts of epidermal keratinocytes: Implications for epidermal function and gene therapy." PROCEEDINGS OF THE NATIONAL ACADEMY OF SCIENCES OF THE U.S.A., vol. 86, no. 22, November 1989, pages 8803-8807, XP000080976 Washington, DC, USA
- E. ERIKSSON ET AL.: "In vivo gene transfer to skin and wound by microseeding." THE JOURNAL OF SURGICAL RESEARCH, vol. 78, no. 2, August 1998, pages 85-91, XP002107026 San Diego, CA, USA
- N. YANG ET AL.: "In vivo and in vitro gene transfer to mammalian somatic cells by particle bombardment." PROCEEDINGS OF THE NATIONAL ACADEMY OF SCIENCES OF THE U.S.A., vol. 87, December 1990 (1990-12), pages 9568-9572, XP001157170 Washington, DC, USA
- R. SANDERS WILLIAMS ET AL.: "Introduction of foreign genes into tissues of living mice by DNA-coated microprojectiles." PROCEEDINGS OF THE NATIONAL ACADEMY OF SCIENCES OF THE U.S.A., vol. 88, no. 7, 1 April 1991 (1991-04-01), pages 2726-2730, XP000368688 Washington, DC, USA
- W. SWAIN ET AL.: "Gene transfer to mammalian cells using AccellTM technology" JOURNAL OF CELLULAR BIOCHEMISTRY, vol. 16, no. F, 1992, page 25, XP008034674

## Description

### Background of the Invention

The invention relates to a use of exogenous DNA in the manufacture of a medicament for providing genetically engineered cells in a patient.

Keratinocytes are the principle cells which cover the surface of the body. They are capable of producing proteins, particularly keratin, which constitutes the main surface barrier of the body. For several different reasons, keratinocytes are attractive potential targets for gene transfer. Since they are located on the surface of the body, they are easily accessed both for gene manipulation and monitoring. If complications from gene transfer would occur, for instance, the development of local tumors or local infections, these could more easily be treated in the skin than elsewhere.

To date, genetic manipulation of keratinocytes has been done in one principal way. Skin has been harvested, the keratinocytes have been separated from the fibroblasts, and then the keratinocytes individually isolated and brought into suspension. These suspensions of keratinocytes have then been cultured to confluence using tissue culture techniques, as reported by Rheinwald, J.G., Green, H. Serial Cultivation of Human Epidermal Keratinocytes: The Formation of Keratinizing Colonies From Cells. Cell G, 331-343, 1975.

The new genetic material has been introduced into the keratinocyte while being grown *in vitro* using either a viral vector or plasmid, as reported by Morgan, J.R., Barrandon, Y., Green, H., Mulligan, R.C. Expression of an Exogenous Growth Hormone Glue by Transplantable Human Epidermal Cells. Science, Vol. 237, 1476-1479 (1987) and Tenmer, J., Lindahl, A., Green, H. Human Growth Hormone in the Blood of Athymic Mice Grafted With Cultures of Hormone-Secreting Human Keratinocytes. FASEB J., 4:3245-3250 (1990). The transfected cells are then usually resuspended and grown on selective media in order to increase the yield of transfection. Sheets of keratinocytes are then transplanted back to the mammal from which the keratinocytes were harvested.

Even though the *in vitro* yield has been acceptable, the *in vivo* yield has been unacceptably low, both short and long term. It has been very difficult to document any significant long term (more than thirty days) expression with these techniques, for example, as reported by Garlick, J.A., Katz, A.B., Fenvjes Esitaichman, L.B. Retrovirus Mediated Transduction of Cultured Epidermal Keratinocytes. J. Invest. Dermatol., 97:824-829, 1991.

It is therefore an object of the invention to provide an improved method for transplanting cells into a patient, especially keratinocytes and genetically engineered cells.

It is a further object of the present invention to provide a means for transplantation which is economical, practical, readily manufactured and can be customized to the patient with minimal effort and expense.

Eriksson et al., Surgical Forum, 1991, Vol 42, pp 670-673 discloses a method for *in vivo* cell culture which accelerates re-epithelialization. WO 87/00201 discloses the preparation of epithelial cells which express foreign genetic material, such as DNA or RNA.

Yang et al., Proc. Natl, Acad. Sci, 1990, Vol 87, pp 9568-9572 discloses *in vivo* and *in vitro* gene transfer to mammalian somatic cells by particle bombardment. Sanders Williams et al., Proc. Natl. Acad. Sci, 1991, Vol 88, pp 2726-2730 discloses the introduction of foreign genes into tissues of living mice by DNA-coated microparticles. Gene transfer to mammalian cells using accelerated microparticles is also disclosed by Swain et al., J Cell Biochem, Supple No 16F, 1992, p 25.

### SUMMARY OF THE INVENTION

The present invention provides the use of an exogenous DNA in the manufacture of a medicament as a solution or suspension for injecting into skin cells of a patient with a microneedle to provide genetically engineered cells *in vivo.*

In a specific embodiment the skin cells are selected from superficial keratinocytes, stem cell keratinocytes and dermal fibroblasts.

The DNA is preferably attached to microparticles, more preferably iron oxide particles from 0.5 to 1 micron in diameter.

In a preferred embodiment the exogenous DNA is injected with a plurality of oscillating microneedles.

The invention further provides a device comprising a plurality of oscillatable microneedles suitable for injection into skin cells, wherein the mirconeedles are loaded with DNA attached to microparticles or in solution or in suspension. Preferably the microparticles are iron oxide particles from 0.5 to 1 micron in diameter.

### Brief Description of the Drawings

Figure 1 is a schematic of the exposed undersurface of a partial thickness skin flap used to expose hair follicles to obtain epidermal stem cells.
Figure 2 is a prospective view of a preferred embodiment of a chamber for culturing keratinocytes on a patient.
Figure 3 is a prospective view of a ported embodiment of a chamber for culturing keratinocytes.
Figure 4 is a schematic representation of a tattooing device for delivery of genetic material into cells.
Figure 5 is a cross-sectional view of a microneedle 10 shown in Figure 4.
Figure 6 is a photograph showing the insertion of genetic material into epidermis cells using a tattooing device.
Figure 7 is also a photograph showing the insertion of genetic material into epidermis cells using a tattooing device.
Figure 8 is a photograph of a histological slide showing keratinocytes in which genetic material has been inserted using the tattooing device.
Figure 9 is a photograph of a histological slide showing keratinocytes in which genetic material has been inserted using the tattooing device.
Figure 10 is a photograph of a histological slide showing keratinocytes in which genetic material has been inserted using the tattooing device.
Figure 11 is a graph showing expression of the gene encoding human growth hormone in keratinocytes, ng/ml versus time in days.
Figure 12a is a photograph of high frequency transfection with lacZ gene, β-Gal positive cells, of primary pig keratinocytes by retroviral gene transfer. Figure 12b is a photograph of expression of the lacZ gene in regenerated epithelium of a full thickness wound reconstituted by retrovirally transfected transplanted keratinocytes, β-Gal staining.
Figure 13 is a graph of protein leakage into wound fluid (mg/dl) over time (days), comparing the efflux of endogenous protein from full thickness wounds in six pigs for normal saline treatment (-squares-) versus keratinocyte suspensions (...triangles...) applied in treatment chambers to the wounds.
Figure 14a is a graph showing continuous production of human growth hormone (hGH) (ng/ml) over time (days) *in vitro* by primary porcine keratinocyte culture after retroviral gene transfer and is a mean of two experiments. Figure 14b is a graph showing human growth hormone (hGH) (ng/ml) over time (days) *in vivo* in wound fluid from full-thickness wounds produced by transplanted suspensions of porcine keratinocytes after retroviral gene transfer. After complete re-epithelialization (histologically, day 6) and formation of a multilayer epithelium, values return to baseline. Mean±SE (n=10)

### Detailed Description of the Invention

U.S.Patent 5,152,757 describes a treatment system for wounds and other skin disorders including a chamber securable about the periphery of a wound, having portal means for introduction into and removal of treatment fluids from the chamber, treatment fluid, at least one treatment additive, control means for treatment variables, and monitoring means for monitoring wound conditions. Treatment fluid and additives are selected according to wound indications and include growth factors, antibiotics, anesthetics, enzymes and oxygen. Treatment variables such as temperature, colloid osmotic pressure, pH, ion concentration and oxygen content are controlled according to wound indications.

The chamber is secured about the periphery of a wound, a treatment fluid and at least one treatment additive introduced into the chamber, and the treatment variables controlled according to wound conditions. Examples of disorders that can be treated include wounds such as burns, pain, tumors, and infections. The chamber can also be used to culture cells onto wounds, where the dispersed cells are introduced into the chamber and allowed to settle and grow at a wound site.

The method described herein is based on two principal components. One is the targeting of epidermal stem cells for gene transfer. The second is the use of the wound chamber in order to create an *in vivo* tissue culture environment, which eliminates the need to culture cells *in vitro* when introducing the genetic material into the cells.

Epidermis keratinocytes are ideally suited for gene transfer and recombinant DNA expression in epidermal wound healing because of their key functions in mechanical stability and integrity and in secretion of biologically active substances, such as apolipoprotein A, which are detectable in the systemic blood circulation. It has been demonstrated that suspensions of genetically engineered keratinocytes can be transplanted onto open wounds, which eliminates the need to grow them into sheets. This makes the process of gene transfer in keratinocytes much easier and less time consuming.

### Cells.

Keratinocytes are separated from the dermis with dispase and then individually separated with trypsin and the fibroblasts removed. By targeting keratinocytes in the hair follicles, which provide a high number of stem cells, it has been found both that the yield of transfection is higher and that these cells are stably incorporated into the basal layer of the epidermis. The cells of the hair follicles serve as "stem cells of the epidermis". By using mechanical separation of the rest of the epidermis from this area these cells can either be removed using a dermatome technique or the genetic material can be directly introduced into these cells *in situ*.

Cells can be obtained as needed from other sites on the patient, including other tissues, for instance, bone marrow cells, muscle cells, and cells from endocrine organs.

### Introduction of Genetic Material

Genetic material can be introduced into the cells by injection with a microneedle. The genetic material would encode proteins such as growth factors, hormones, and other therapeutic proteins. These would speed the healing of wounds and correct certain deficiencies, such as parathyroid, growth hormone, and other hormone deficiencies, as well as deficiencies of certain clotting factors such as factor VIII. Cells can also be engineered to not express a protein, such as a protein involved in an immune response, for example, a human leukocyte antigen (HLA).

Keratinocytes are harvested and brought into suspension through exposure to enzymes, first to dispase and then trypsin, then grown to subconfluence in an incubator. Recombinant DNA is then introduced into the cell using a viral vector, plasmid or gene gun technique. After this the keratinocytes are suspended and injected into full thickness wounds which are covered by a wound chamber. These keratinocytes will not only survive, but will show continued presence of cells expressing the marker gene in the basal layer of the epidermis.

The keratinocytes and fibroblasts in the wound can be made to produce, for instance, growth factors, if the gene encoding the growth factor is introduced into the genome or cytoplasm of these cells. The expressed growth factor will then not only speed the healing of the wound, but also help to heal wounds that would not heal otherwise. The genetically engineered keratinocytes can therefore be used to deliver growth factors into the wound in a way that is determined by the wound environment itself.

This approach can be used to insert other genes of interest into keratinocytes in order to reinstitute functions defective in a variety of skin diseases. Also, the mode in which cytokines act can be changed in that a factor is expressed in a cell that normally does not produce that factor, thus changing a paracrine into an autocrine pathway. Instead of native cells, genetically engineered keratinocytes can be seeded and can secrete new protein into the wound during the healing process. In the same fashion, keratinocytes can be genetically engineered to repair inherent genetic defects, such as epidermolysis bullosa. These cells can then be seeded onto a superficial excisional wound to generate a new epidermis with the desired features. Problematic wounds require coverage of the defect and fast healing.

### The Chamber

In the preferred embodiment, cells are cultured in a system including a chamber, treatment fluid (which may be nutrient media, physiological saline, or some other compatible solution), treatment additives (such as antibiotics and buffering agents), means for controlling treatment variables and means for monitoring cell growth.

The wound chamber, which is made of vinyl or other flexible transparent material, such as polyurethane, has a bellows shape and an opening which corresponds to the size of the wound, either the chronic wound or a superficial wound created specifically for the purpose of gene transfer. The chamber contains a small amount of normal saline with antimicrobial agents. When plasmid or retroviral vectors are used, the chambers are simply attached over the wound and the vector is injected into the chamber and the chamber is then resealed. When the gene gun is used, the DNA is delivered into the cell and the chamber with normal saline and antibiotics is then attached to the perimeter of the wound. Wound fluid is sampled in order to assay expression of the secretable gene product (growth factor) at 24 and 48 hours. The wound is treated in the chamber until healed.

The chamber encloses a predetermined surface area about the culture site. The chamber provides protection for the wound from the surrounding non-sterile environment, control of treatment variables, containment for continuous fluid treatment, an effective delivery system for additives, direct monitoring of cell growth. Monitoring can be accomplished visually if the chamber is formed of a transparent material, or by extraction and analysis of fluid from the chamber. Fluid extracted from the system can be analyzed for factors which provide an indication of the status of healing, as well as the presence of undesirable components such as microorganisms, low oxygen, high carbon dioxide and adverse pH. The fluid may be tested for the number and type of bacteria and other microorganisms, the number and type of cells, the amount and type of proteins secreted by the patient and the cells within the chamber, and other factors such as drug levels, oxygen, carbon dioxide and pH.

The treatment system provides control over variables including temperature, specific ion concentration, colloid osmotic pressure, glucose concentration, amino acid content, fat concentration, oxygen concentration and carbon dioxide concentration and pH.

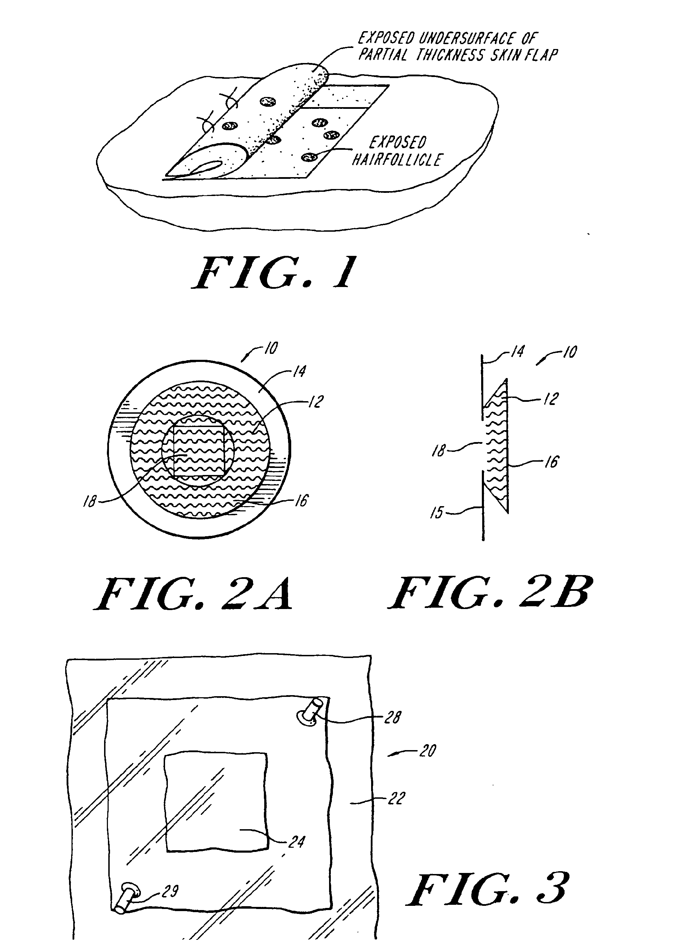

Portal means provide access for the introduction of treatment fluids and treatment additives into the chamber and extraction of fluid from the chamber. In some embodiments such as that shown in Figure 2, treatment fluid is introduced into the chamber by injection with a conventional hypodermic syringe through the wall of the chamber, preferably made of a flexible, self-repairing plastic. In other embodiments such as that shown in Figure 3, the chamber has an inlet and outlet port or separate inlet and outlet ports. Valve mechanisms are necessary where the apparatus is not to be connected to a treatment fluid reservoir and a drain or connected to a continuous perfusion system. The seals of the ports would be broken at an appropriate time for connection to other apparatus, such as a continuous perfusion system, at a hospital for example.

A preferred embodiment of the treatment system incorporates continuous perfusion of treatment fluid through inlet and outlet ports. A pump or gravity may be used to move the treatment fluid. The treatment fluid may be recirculated after filtering and other appropriate action (eg. heating or cooling). Alternately, fresh treatment fluid may be introduced and contaminated fluid disposed of. In an embodiment described in U.S. 5,152,757, the chamber contains a reservoir or more than one chamber, with the additional chamber serving as a source of fresh culture media, oxygen, and treatment additives.

Figure 2a and b show a preferred embodiment of a chamber 10, Figure 2a is a top view and Figure 2b is a side view. The chamber 10 has a bellows-type construction that prevents the majority of the chamber from contacting the wound service and in fact acts to push the chamber away from the wound. The chamber 10 is preferably constructed of clear vinyl or other sterilizable, flexible, heat-sealable, gas and moisture impermeable material. The bellows fold 12 allows for a large capacity for treatment fluid as well as ease of construction. For construction, an annular base 14 is heat or ultrasound welded to an annular bellows fold 12 piece and a circular top 16 welded to bellows fold 12 piece. Circular top 16 is a substantially transparent material. The remaining components are not necessarily transparent but may be. A transparent annular base may provide easy visual inspection for leakage. An opening 18 in annular base 14 is provided that corresponds in size to the wound or site for transplantation. The bottom side 15 of annular base 14 is provided with an adhesive tape or coating suitable for securing the chamber to skin.

As those skilled in the art will readily recognize, a removable sheet for protecting the adhesive and maintaining the sterility of the interior of the chamber is desirable. The chambers may be stored in a sterile pack for years. This chamber can take many shapes in order to fit wounds from the size of one square centimeter up to the size of a whole extremity. It is important that the adhesive surface be sufficient to secure the bandage to the skin surface to ensure a leak-proof seal.

As previously mentioned, treatment fluid and treatment additive introduction and subsequent extraction may be accomplished directly through the chamber walls by a needle and syringe. A self-repairing material to construct chamber 10 is contemplated. An alternative method would be to use inlet and outlet ports allowing the introduction and extraction of various substances into the chamber.

Figure 3 is a view of a chamber 20 for enclosing a predetermined surface area about a site on a patient where cells are to be cultured, where the chamber is open to the skin 24 and sealed at the edges 26 to the skin surface about the wound by means of an adhesive. The chamber 20 has a transparent section 22 for visual wound monitoring, a portal means 28, 29 for introduction of treatment fluid and treatment additives into the chamber and extraction of fluid from the protective chamber, and monitoring means (not shown) for analyzing extracted fluid for predetermined variables and wound conditions.

### Direct introduction of the genetic material into keratinocytes.

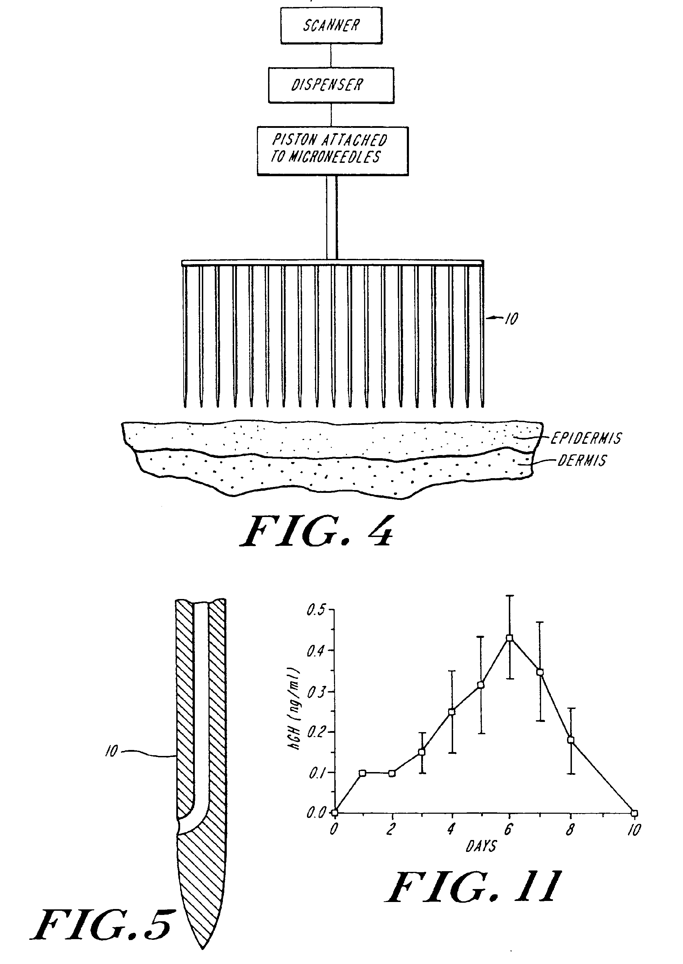

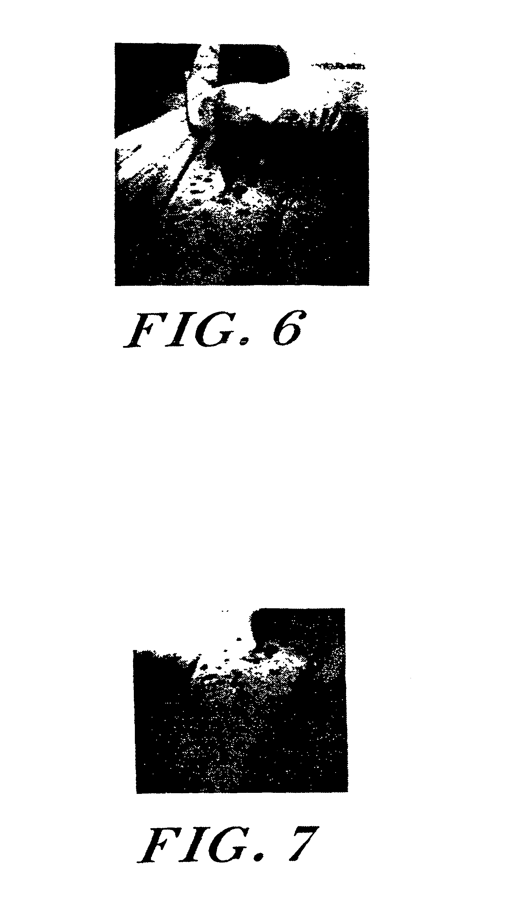

A crossbreeding of tattooing and microinjection is used to insert DNA material directly into cells. As shown in Figure 4, a plurality of microneedles 10 are mounted in a row on a handle. The microneedles 10 are pointed and hollow as shown in Figure 5. A solution containing genetic material, preferably DNA, is placed on the epidermal surface. The needles 10 are then moved as a group across the skin to "inject" and thereby deliver the genetic material through the surface of the skin to the underlying epidermis along several parallel lines. Microneedles such as tattooing needles are ideal for this purpose. Preferably, the needles are used to deliver microparticles, such as iron oxide microparticles having DNA material attached to them, as shown in Figures 6-10.

Alternatively, a row of microneedles is attached to a central dispenser containing a solution of microparticles to which the DNA material is attached and the solution is injected into skin cells, such as keratinocytes. In addition, this technique is useful for delivery of a suspension of DNA material alone, without attachment to microparticles.

This system, attached to a scanner, could systematically cover a specific area of skin or other tissue and deposit the DNA material very evenly into a large number of cells in the predefined area.

This method of introducing genetic material into keratinocytes may be superior to gene transduction using plasmids or retroviral vectors because the latter have an unacceptably low yield. Transfer of genetic material with accelerated particles, employing "gene guns", have a higher yield than plasmid or retroviral transduction, but have other disadvantages, such as a very loud blast and the risk of accidental discharge.

This combined technique of tattooing and microinjection of genetic material into cells with oscillating microneedles provides a practical, affordable and yet also a predictable method of cellular insertion of DNA material for systemic expression.

### Preparation of site for culturing cells.

The site for culturing cells is prepared by removing infected or burned skin, if necessary, or by creating an appropriate wound for transplantation of engineered cells. The skin adjacent to the wound is then cleaned so that there will be good adhesion between the chamber and the skin. The open portion of the chamber is then placed over the site, with the adhesive edges securing the chamber to the skin, then an appropriate culture medium and cells are introduced into the sealed chamber. The treatment fluid may be introduced and then extracted in favor of fresh culture media in a continuous or batch process. Selected treatment additives may be introduced into the chamber continuously or at a predetermined time or at periodic intervals. Appropriate control of treatment variables is also effected. Monitoring is accomplished by examination of the patient and visual examination of the fluid within the chamber and the wound itself. In addition, samples of fluid are extracted from the chamber for analysis and diagnosis. The chamber is removed once sufficient healing of the wound has occurred.

For example, to determine whether or not the wound is healed, the protein content of the extracted fluid is analyzed. When the protein content of the extracted fluid decreases to the level present in chambers containing fluid that are placed over normal skin, the wound is healed. Methods for determining protein content is well known in the art and are inexpensive and fast. The types of protein and the relative amounts of the types of protein can also be determined to further evaluate healing and expression of exogenous genetic material.

### Control of Treatment or Culture variables.

Treatment of each patient is specific for the conditions within the chamber. Control over treatment variables can include continuous cooling to 34°C for the first 24 hours. Monitoring can include analyzing extracted fluid for protein and microorganisms, with samples extracted every 24 hours. For example, when the number of microorganisms is less than 10 to the 4th per milliliter or per cc, infection has been resolved. Protein levels checked every day should be less than 24 mg/dl/cm².

As noted above, there are a number of treatment variables which may be controlled by the system. One such treatment variable which may be controlled is temperature. It has been found that heating the wound from a temperature of approximately 27°C (a common temperature of a lower extremity wound) to 37°C accelerates wound healing. Experimental data has shown that at a wound temperature of approximately 37°C, the rate of wound healing is more than twice as fast as at a temperature of 27°C. The temperature of the wound area can be achieved by heating the treatment fluid. Cooling has also been proven beneficial in the case of acute burn and other traumatic wounds. Cooling reduces pain, swelling and destruction of tissue. In general terms, acute wounds benefit from cooling during the first hours after occurrence of the wound and later, wounds benefit from a temperature of approximately 37°C. Cooling can similarly be effected by cooling the treatment fluid.

Other treatment variables may also be optimized. For example, ion concentrations should be kept close to extracellular ion levels. Glucose, amino acid and fat concentrations should be kept close to the concentrations present in plasma or corresponding to a skin tissue culture medium. Oxygen and carbon dioxide concentrations should also be maintained at their normal tissue levels. Oxygen is an important treatment additive, and is essential for cell growth.

### Treatment additives and Culture Media.

Normal or physiological buffered saline is the basic culture media. Buffering agents, anesthetics such as lidocaine, antibiotics such as penicillin or streptomycin, chemotherapeutic agents, and growth factors including epidermal growth factor (EGF), platelet-derived growth factor (PDGF), insulin-like growth factor (IGF), basic fibroblast growth factor (bFGF), and cholera toxin (CT) can be added to the culture/treatment media. Tissue culture mediums and fluids which increase osmotic pressure and oxygen accessibility may also be introduced to the chamber as treatment additives.

Selection of treatment additives is wound specific. For example, if an infection has been diagnosed, antibiotics are added in the amount of one single parenteral dose per 1,000 cc of fluid. Furthermore, a treatment additive of gentamicin, tobramycin or carbenicillin is appropriate for a wound infection with *Pseudomonas*, detected by analyzing extracted fluid. When hypoxia has been diagnosed, the liquid is passed through an oxygenating chamber before entering the chamber. If a tumor has been diagnosed, chemotherapy is given in an amount of one single parenteral dose per 1,000 cc of fluid. In situations involving a wound containing necrotic tissue and debris, proteolytic enzyme is added to the liquid. Immune modulators are added to the treatment fluid if an inflammatory reaction is exhibited. Epidermal growth factor is added in a concentration of 10 nanograms per cc when required.

The present invention will be further understood be reference to the following non-limiting examples.

### Example 1: Transfection of keratinocytes and expression of human growth hormone in culture and following transplantation into partial thickness wounds.

In the first series of experiments, keratinocytes were harvested, plated in culture flasks, and transfected with a viral vector technique, using the method of Wilson, J.M, Biriuyi, L.U., Salomon, R.U., et al. Transplantation of Vascular Grafts Lined With Genetically Modified Endothelial Cells. Science, 244:1344-1346 (1980), or a plasmid transfer technique, as described by Felgner, P.L., Galik, T.R., Holmer, et al. Lipofection: An Efficient, Lipid Mediated DNA-Transfection Procedures. Proc. Natl. Acad. Sci., 84:7413-7417 (1987), the teachings of which are incorporated herein.

Pigs are the standard animal model for extrapolation to human skin because porcine skin resembles human skin in several respects, including turnover time.

Specifically, keratinocytes were harvested from two female Yorkshire pigs by partial thickness skin excision, dispase separation of epidermis (0.25% Dispase, 2-3 h at 37°C), trypsinization (0.1% Trypsin, 0.02% EDTA, 30 min at 37°C) and mincing to a single cell suspension. They were grown to subconfluence (60-70%) and incubated with a replication defective murine Moloney leukemia retroviral vector. The DNA sequence for hGH was inserted into the viral genome and was under the promoter control of the viral long-terminal repeats (LTR's). The infected keratinocytes were trypsinized, resuspended in saline containing penicillin (100 U/ml) and streptomycin (100 µg/ml). They were transplanted to full thickness wounds created on the dorsum (n=10) in an autologous fashion. Full thickness wounds seeded with unmodified keratinocytes (n=10) and saline (n=10) served as controls. All wounds were covered with sterile vinyl chambers for maintenance of a liquid wound environment throughout the study. The presence of hGH in the culture medium or the wound fluid was determined by radioimmunoassay.

The same methodology was used with the lacZ gene, an intracellular marker gene.

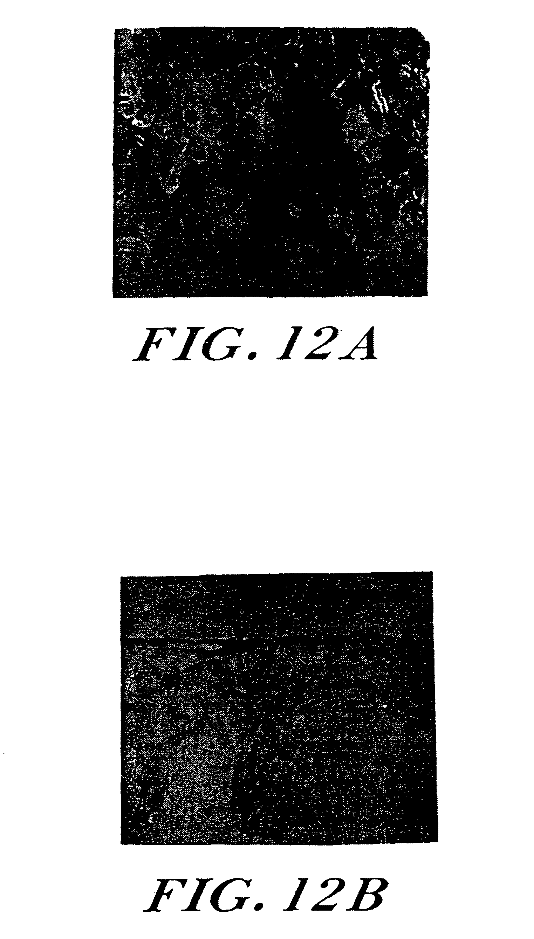

Significant expression of both genes in this system was obtained, as shown by Figures 11 and 12a and b. Histologic sections of the skin showed that the keratinocytes that had the lacZ marker gene were not stably incorporated into the basal layer, but migrated to the surface and were lost into the stratum corneum. When analyzing the reasons behind this, it was concluded that the dispase separation of dermis from epidermis did not allow for harvesting of a sufficient amount of stem cells. An additional reason was that the repeated trauma of harvesting the cells, exposure to tissue culture medium with relatively high calcium, and the cellular injury from the virus or the plasmid medium, caused a near terminal differentiation of the keratinocytes. Accordingly, results were greatly improved by using a higher percentage of epidermal stem cells obtained from the hair follicles, and decreasing the exposure to calcium and viral or bacterial medium.

*In vitro* (culture medium) hGH was detected on day 4 after retroviral transfection with a mean daily production of 2.0 ng/ml. *In vivo* (wound fluid) hGH production was first detected 24 hours after transplantation (0.1 ng/ml) peaked at 132 h (0.42 ng/ml) and returned to baseline by day 10. Healing times, assessed by the protein efflux assay in wound fluid, wounds receiving hGH-keratinocytes (12.1 ± 1.0 days) were no different than those of wound receiving non-infected keratinocytes but significantly shorter (p < 0.005) than non-transplanted control wounds (14.7 ± 0.6 days). Histologically transplanted keratinocytes established a multilayer epithelium as early as day 6 (saline treated controls by day 11).

The results demonstrate that autologous keratinocytes carrying the hGH gene will continue to express the hGH gene when transplanted to full thickness wounds. The produced hGH does not influence healing times. Compared to the *in vitro* keratinocyte cultures hGH production *in vivo* is regulated in a time dependent fashion. This may be either due to a regulation of hGH gene expression by keratinocytes during epithelialization, loss of hGH producing keratinocytes due to terminal differentiation, or antibody reaction to the foreign protein, as discussed by Selden, et al., "Human Growth Hormone as a Reporter Gene in Regulation Studies Employing Transient Gene Expression" Mol. Cell Biol. 6(9):3173-3179 (1986). Since genetically engineered keratinocytes not only improve re-epithelialization of full thickness wounds but also release a recombinant DNA product during healing they can be used to deliver and express therapeutic peptides, like growth factors, during wound healing. By using a topical chamber system, direct application of genetically engineered cells and monitoring of the gene product can easily be facilitated.

### Example 2: In Vivo Culture of Genetically Modified Keratinocytes.

Unmodified and retrovirus-transduced keratinocytes expressing the β-galactosidase gene and human growth hormone gene (hGH) were transplanted into enclosed skin wounds of Yorkshire pigs. Analyses of sequential skin samples obtained by biopsy during the four weeks after transplantation revealed survival of the transplanted cells and expression of β-galactosidase. Transfected keratinocytes were first documented in the deep portions of the wounds and then in the basal layer of regenerated epidermis. These cells contributed to the normal skin architecture and barrier function and transferred the new gene product to terminally differentiating cells of the stratum spinosum. When keratinocytes transduced with the hGH gene were transplanted into wounds, hGH was detected for up to 10 days in wound fluid. In contrast, hGH production *in vitro* was continuously detectable for 47 days. The pattern of expression demonstrates that transduced keratinocytes reconstitute the new epidermis and undergo terminal differentiation and could be used for the introduction and expression of therapeutic proteins in skin disease to promote skin replacement and healing.

Porcine wounds (partial and full thickness burns, incisions and excisions) and human wounds (chronic full thickness) were individually enclosed in vinyl chambers containing placebo fluid with additives. Unless otherwise specified, the placebo fluid was unbuffered 0.9% NaCl with 100 IU of penicillin/ml and 100 µg of streptomycin/ml. Various media for the culture of keratinocytes were also used. Maintenance of the chamber system took place twice daily, daily, every other day, or continually. The wounds were treated in the chambers until healed. The wounds were assessed macroscopically, biochemically (by analysis of the chamber fluid) and microscopically. Both wound fluid and human growth hormone (hGH) in vitro culture fluid were assessed in the Allegro^{™}. Radio-Immuno-Assay (Nichols Institute Diagnostics, San Juan Capistrano, CA)

The β-galactosidase gene of *Escherichia coli* (lacZ) was inserted into porcine keratinocytes through transduction with a murine Moloney leukemia vector in which the β-galactosidase gene is under control of the retrovirus long terminal repeat, as described by Danos et al., Proc. Natl. Acad. Sci. 85:6560-6464 (1988). Suspensions of native or retrovirally transduced keratinocytes were transplanted into surgically-created full-thickness skin wounds in 13 female yorkshire pigs with a vinyl chamber as a temporal *in vivo* incubator. Porcine skin resembles human skin in several respects (e.g. turnover time) and therefore was the most appropriate model for this study. Domestic female Yorkshire pigs (3-4 months old; 40-45 kg) were used. Primary keratinocyte cultures were established from partial thickness skin grafts (0.012" thick) taken from the dorsal neck region of the pigs under general inhalational anesthesia. Sections were incubated in serum free medium containing 0.25% Dispase^{™} at 37°C for 2 to 3 hours to separate the epidermis from the dermis. A single cell suspension of keratinocytes was obtained by incubation in 0.1% trypsin and 0.02% EDTA (37°C for 30 minutes) and careful mechanical disruption. The solution was filtered through a 100 µm mesh and resuspended in medium. Cells were then seeded into culture flasks at a density of 0.12 x 10⁶ cells/cm² and grown in Weymouth medium containing 20% fetal bovine serum.

At 60-80% confluence keratinocyte cultures assigned for gene transfer were transfected with an amphotropic helper-free murine leukemia virus (ψ-CRIP) containing the β-galactosidase gene (lacZ). Subconfluent cell cultures were incubated on three consecutive days with the retrovirus-containing medium. Unmodified keratinocytes were grown to confluence without manipulation.

A total of 170 full thickness wounds were created on the backs of 13 pigs, with the panniculus carnosus muscle left intact. After careful hemostasis a liquid-tight vinyl chamber serving as an *in vivo* cell culture device was applied to each wound. The chamber (P.A. Medical Corp., Columbia, TN) consists of a flexible transparent vinyl top bonded to an adhesive base. The base has a central opening compatible with the margin of the created wound. Chambers were filled with 1.2 mL of normal saline containing 100 µg streptomycin and 100 IU penicillin/ml (n-67), transduced skin keratinocytes (lacZ, n=22 hGH, n=10) or nontransfected keratinocytes (controls, n=71). Starting on day 2 (to give the cells time to attach to the surface) the saline chambers were exchanged and refilled on a daily basis.

The effects of transplantation of keratinocytes on re-epithelialization were studied macroscopically, histologically, and by measurement of endogenous proteins in chamber fluid (as a noninvasively evaluable parameter of epithelial barrier function). Fluid collected from individual wounds was centrifuged at 2,000 x g for 10 minutes and then passed through a filter (pore size 0.45 mm, Millipore, Corp., Bedford, MA). Endogenous proteins as a noninvasive marker of restoration of epithelial barrier function was measured with a turbidimetric assay (STANBIO CSF, Stanbio Laboratory Inc., San Antonio, TX) as described previously by Breuing et al., J. Srug. Res. 52:50-58 (1992).

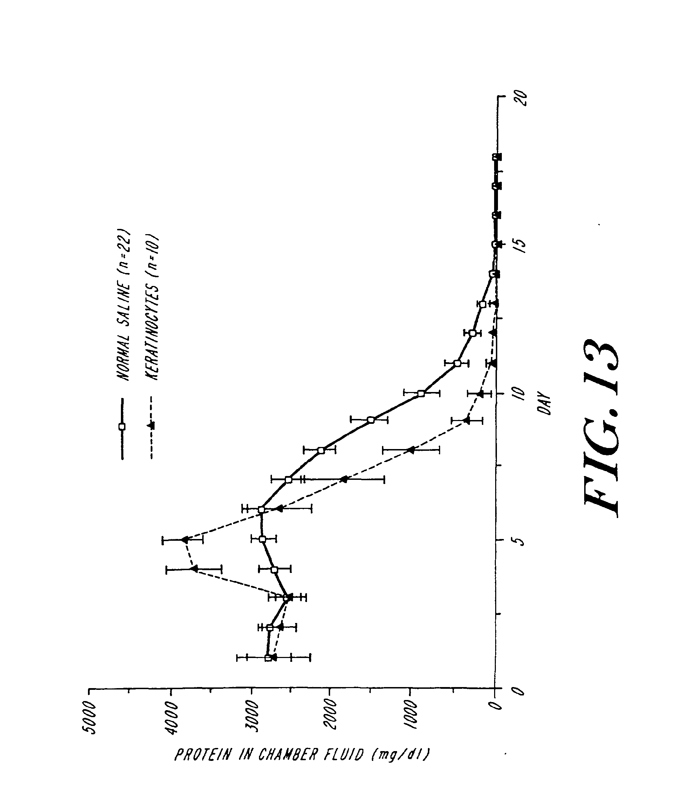

Chamber-treated wounds developed no tissue necrosis while wounds covered with conventional dressings were significantly necrotic. This difference was visible macroscopically. After 4 days a fibrinous clot had developed in chamber treated wounds of all groups. From day 6 on, wounds into which either unmodified or retrovirus-transduced keratinocytes had been transplanted were covered with a thin epithelium; this development was not seen in saline controls until day 12 as shown in Figure 13. Wound contraction did not differ in the three groups.

The macroscopic appearance of porcine full thickness wounds on days 3, 6, and 9 after wounding were examined. At confluence, keratinocytes were released from flasks with 0.01% trypsin and resuspended in normal unbuffered saline (0.9%) containing 100 U penicillin and 100 µg streptomycin/ml, at a concentration of 3 x 10⁶ cells/ml. A 1.2 ml volume of this suspension was injected into full-thickness wounds (15 x 15 mm, 9 mm deep) created on the pig dorsum. Keratinocytes resuspended in saline were found to be more than 90% viable, as confirmed by trypan blue uptake and reculturing of the content of individual syringes in culture flasks. Means and standard errors were calculated for the groups. Statistical significance between groups was analyzed with the nonparametric Mann-Whitney U-test. A logistic regression analysis of the reestablishment of the neoepidermis, as evaluated histologically, was performed.

Wounds into which transplanted keratinocytes were transplanted developed an early epithelium on day 6, while saline treated controls still contained a clot that was not re-epithelialized clot. The rate of wound contraction was not affected. Therefore, the epithelial barrier was established significantly earlier after transplantation of suspensions of autologous keratinocytes than after treatment with saline only (mean ±SD 12.66±16 days versus 14.7±.7 days; p < 0.05).

This difference was confirmed histologically by documentation of complete epithelialization on day 12 after transplantation but only on day 16 after treatment with saline. In wounds with transplanted keratinocytes, the following characteristic pattern was observed. Retrovirus transduced, lacZ-expressing keratinocytes first appeared in clusters on the bottom of the wounds on day 4 and then migrated upward with the newly developing matrix, as seen on day 6. By day 8 they were present in all layers of epithelium. Therefore, clusters of these cells were first seen on day 4 on the deep wound surface and later in the developing matrix. These clusters were evident until a new epithelium developed and disappeared thereafter.

*In vitro* staining of wounds with transplanted lacZ-positive keratinocytes revealed β-galactosidase positive cells within clusters of colonies on the bottom of the wound, closely related to the subcutaneous tissue on day 4. On day 6, the cells appeared in the center of the newly-developing connective tissue. Serial sections stained with hematoxylin and eosin and with Masson's trichrome showed that these nests were surrounded by dense collagen bundles. By day 8 lacZ-expressing keratinocytes were distributed in all cell layers of the newly regenerated epithelium including the stratum germinativum. Wounds biopsied at 27 days showed lacZ expression only in the upper portion of the stratum granulosum and not in the stratum basale or the stratum spinosum. Cross-sectional biopsy samples including margins of unwounded skin were taken from the wounds from day 4 to day 27. Half were fixed in 10% formalin, embedded in paraffin and stained with hematoxylin/eosin, or trichrome. The other half were snap-frozen in liquid nitrogen, sectioned, and stained for the detection of β-galactosidase-activity *in situ* with the substrate 5-bromo-4-chloro-3-inolyl β-D-galactoside (X-Gal chromogen), which forms a blue precipitate in transduced cells. By day 27, no additional nests of keratinocytes were detected in the subepidermal tissue.

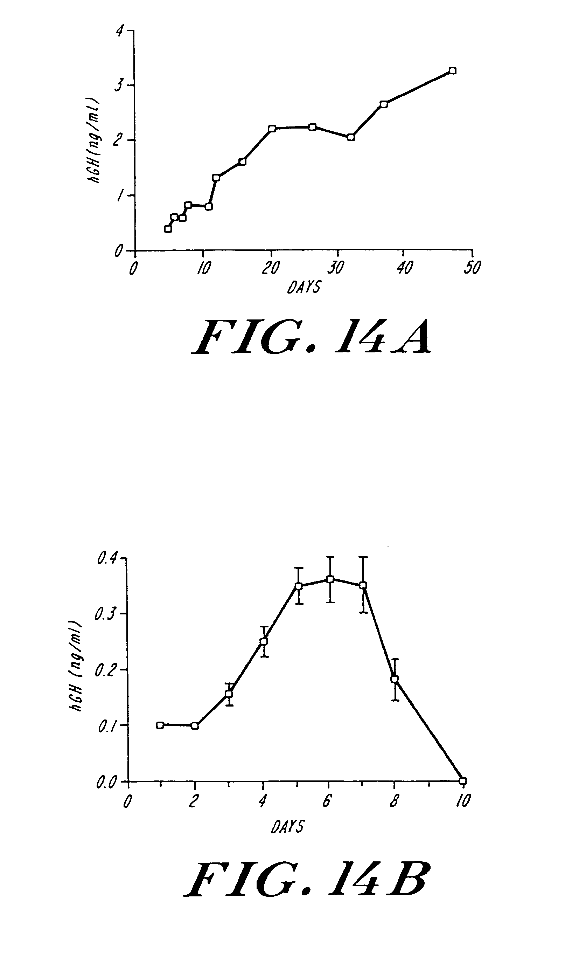

The histological appearance of the epithelium after transplantation of either transduced or unmodified keratinocytes was similar and was characteristic of a cornified multilayer epithelium. Horizontal sections through the epidermis showed evidence of an even distribution of transduced cells (280/mm²). Keratinocytes transfected with both *lacZ* and hGH (n=34 wounds) transplanted into the wounds produced hGH in a temporary fashion, with concentrations peaking at day 6 and declining to 0 until day 10 as shown in Figure 14b.

This experiment demonstrates that suspensions of keratinocytes can be stably transduced with a gene and that these cells continue to express the gene after transplantation into full thickness skin wounds in pigs. In a cutaneous-chamber system, the cells were successfully transplanted, survived, and proceeded from cluster formation to reconstitution of new epithelium in a predictable fashion. The epidermis stratified and formed a functionally intact epithelial barrier. The wet environment of this closed-chamber system creates a favorable environment for the transplantation of cells. It includes all the necessary components such as serum electrolytes at physiologic osmolality and pH and a variety of growth factors (e.g. basic fibroblast growth factor epidermal growth factor) that exhibit mitogenic activity for keratinocytes.

The decreasing expression of lacZ may be explained by an early and rapid terminal differentiation of the transduced keratinocytes, which is likely when the specific pattern of lacZ expression is taken into account. The high calcium concentration in fluid as well as the presence of other mediators and the interactions of these mediators with cells in the wound may induce terminal keratinocytes differentiation. The morphologic and functional formation *in vivo* of an epithelial barrier of lacZ-expressing keratinocytes indicates that the cells have stratified and terminally differentiated. This conclusion is supported by the finding of lacZ-expressing keratinocytes only in the uppermost stratified layers of the epithelium after 27 days. Therefore, this experiment shows that suspensions of retrovirus transduced keratinocytes form a multilayered epithelium *in vivo* after autologous transplantation into full thickness wounds. Moreover, these cells provide function in a manner similar to unmodified keratinocytes in that they stratify and form an epithelial barrier to water and protein loss. Thus it is possible to regenerate a new epithelium with physiological properties through the use of genetically manipulated-keratinocytes.

### Example 3: Introduction of DNA into keratinocytes and fibroblasts using "tattooing".

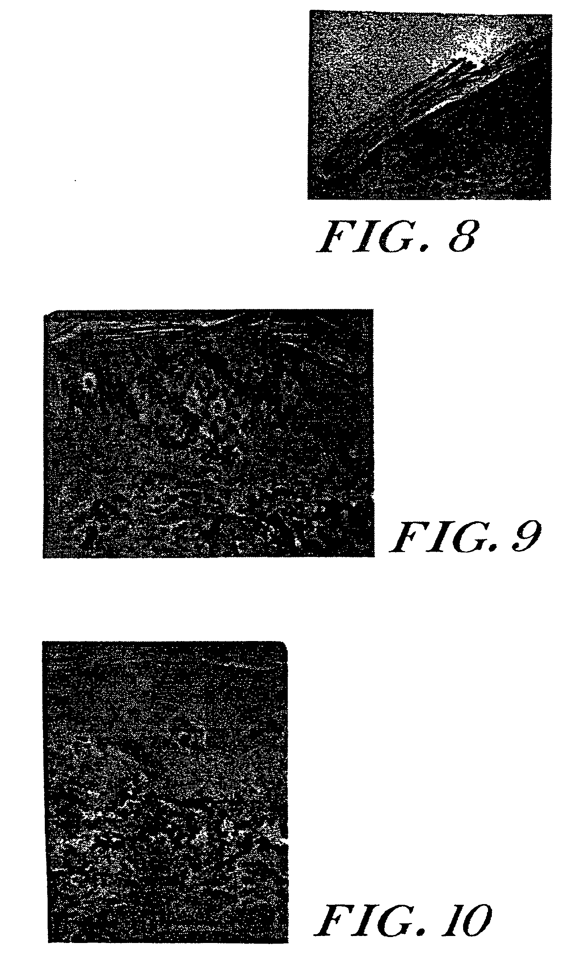

Iron oxide particles ranging in size from 0.05 microns to 1 micron in diameter were mixed with DNA plasmids in Tris-EDTA buffer. Drops of this material were placed on intact human skin. A tattooing device, containing microneedles, was placed on the material on the skin to insert, or inject, the DNA into superficial keratinocytes as shown in Figure 6, as well as stem cell keratinocytes in the deep epidermis, as shown in Figure 7, or dermal fibroblasts, as shown in Figure 8.

## Claims

1. Use of an exogenous DNA in the manufacture of a medicament as a solution or suspension for injecting into skin cells of a patient with a microneedle to provide genetically engineered cells *in vivo.*

2. Use according to claim 1 wherein the skin cells are selected from superficial keratinocytes, stem cell keratinocytes and dermal fibroblasts.

3. Use according to claim 1 or 2 wherein the exogenous DNA is attached to microparticles.

4. Use according to claim 3 wherein the microparticles are iron oxide particles from 0.5 to 1 micron in diameter.

5. Use according to any one of the preceding claims wherein the exogenous DNA is injected with a plurality of oscillating microneedles.

6. A device comprising a plurality of oscillatable microneedles suitable for injection into skin cells, wherein the microneedles are loaded with DNA attached to microparticles or in solution or in suspension.

7. A device according to claim 6, wherein the microparticles are iron oxide particles from 0.5 to 1 micron in diameter.

## Patentansprüche

1. Verwendung einer exogenen DNA für die Herstellung eines Arzneimittels als eine Lösung oder Suspension zur Injektion in Hautzellen eines Patienten mit einer Mikronadel, um in vivo gentechnisch veränderte Zellen bereitzustellen.

2. Verwendung nach Anspruch 1, wobei die Hautzellen ausgewählt werden aus oberflächlichen Keratinozyten; Stammzell-Keratinozyten und Hautfibroblasten.

3. Verwendung nach Anspruch 1 oder 2, wobei die exogene DNA an Mikropartikel gebunden ist.

4. Verwendung nach Anspruch 3, wobei die Mikropartikel Eisenoxidpartikel mit einem Durchmesser von 0,5 bis 1 Mikrometern sind.

5. Verwendung nach einem der vorhergehenden Ansprüche, wobei die exogene DNA mit mehreren oszillierenden Mikronadeln injiziert wird.

6. Vorrichtung, umfassend mehrere Mikronadeln, welche oszillieren können und zur Injektion in Hautzellen geeignet sind, wobei die Mikronadeln mit DNA, welche an Mikropartikel gebunden ist, oder in Lösung oder in Suspension vorliegt, beladen sind.

7. Vorrichtung-nach Anspruch 6, wobei die Mikropartikel Eisenoxidpartikel mit einem Durchmesser von 0,5 bis 1 Mikrometern sind.

## Revendications

1. Utilisation d'un ADN exogène pour la fabrication d'un médicament tel qu'une solution ou une suspension pour injecter dans des cellules de peau d'un patient avec une microaiguille afin de fournir des cellules génétiquement modifiées *in vivo.*

2. Utilisation selon la revendication 1, dans laquelle les cellules de peau sont choisies parmi les kératinocytes superficiels, les kératinocytes de cellules souches et les fibroblastes dermiques.

3. Utilisation selon la revendication 1 ou 2, dans laquelle l'ADN exogène est attaché à des microparticules.

4. Utilisation selon la revendication 3, dans laquelle les microparticules sont des particules d'oxyde de fer de 0,5 à 1 micron de diamètre.

5. Utilisation selon l'une quelconque des revendications précédentes, dans laquelle l'ADN exogène est injecté grâce à un ensemble de microaiguilles oscillantes.

6. Equipement comprenant un ensemble de microaiguilles oscillantes adaptées à l'injection dans des cellules de peau, équipement dans lequel les microaiguilles sont chargées avec de l'ADN attaché à des microparticules ou en solution ou en suspension.

7. Equipement selon la revendication 6, dans lequel les microparticules sont des particules d'oxyde de fer de 0,5 à 1 micron de diamètre.
